# EUROPEAN PATENT APPLICATION

(11) **EP 1 674 087 A1**
(43) Date of publication of application: **28.06.2006**
(21) Application number: 06100160.8
(22) Date of filing: 28.09.2001
(51) Int. Cl.: A61K 31/00, A61K 31/451, A61K 31/453, A61P 25/00, A61P 25/02, A61P 25/04, A61P 25/06, A61P 29/00

(54) **Prophylactic use of n-methyl-d-aspartate (NMDA) antagonists**

(30) Priority: 02.10.2000 US 237324 P
(62) Divisional of application: 01308289.6
(71) Applicant: Pfizer Products Inc., Groton, Connecticut 06340 (US)
(72) Inventor: Chenard, Bertrand Leo, Groton, CT 06340 (US); MennitiI, Franck Samuel, Groton, CT 06340 (US); Saltarelli, Mario David, Groton, CT 06340 (US); Schneider, Erika, Groton, CT 06340 (US)
(74) Representative: Lane, Graham Mark Hamilton

(57) **Abstract**

The invention relates to the use of an NR2B subunit selective NMDA antagonist in the manufacture of a medicament for preventing primary hyperplasia, secondary hyperplasia, primary allodynia, secondary allodynia, or other pain caused by central sensitization, in a mammal, characterised in that the medicament is administered prior to affliction with said pain.

## Description

### Field of the Invention

This invention relates to inhibition of neurological damage. In particular, this invention relates to a method of inhibiting in a mammal, for example a human, neurological damage associated with ischemic injury comprising administering to the mammal, prior to an event having associated therewith risk of ischemic injury, an effective amount of an NR2B subunit selective N-methyl-D-aspartate (hereinafter NMDA) antagonist. This invention also relates to prevention of pain caused by central sensitization in mammals, including humans. In particular, this invention relates to a method of preventing central sensitization in a mammal, such as a human, comprising administration of an affective amount of an NR2B subunit selective NMDA receptor antagonist.

### Background of the Invention

### NMDA Receptors and NMDA Receptor Subunits

Glutamate and aspartate play dual roles in the central nervous system as essential amino acids and as the principal excitatory neurotransmitters (hereinafter referred to as excitatory amino acids or EAAs). There are at least four classes of EAA receptors: NMDA, AMPA (2-amino-3-(mothyl-3-hydroxyisoxazol-4-yl)propanoic acid), kainate and metabotropic receptors. These EAA receptors mediate a wide range of signaling events that impact all physiological brain functions. For example, it has been reported that NMDA receptor antagonists produce an analgesic effect under certain conditions (Wong, C.S., Chemg, C.H. and Ho, S.T., Clinical Applications of Excitatory Amino Acid Antagonists in Pain Management *Acta Anaesthesiologica*.*Sinica*; 33, 227-232 (1995)).

The NMDA receptor is an ion channel permeable to Na⁺ and Ca²⁺. The receptor is gated by synaptically released glutamate in the presence of co-agonist glycine and concomitant depolarization (Mayer, M.L. and Westbrook, G.L., The Physiology of Excitatory Amino Acids in the Vertebrate Nervous System, Progress *in Neurobiology*, 28, 197-276 (1987)). Thus, NMDA receptor activity may be attenuated by blockade, for example, of 1) the glutamate binding site, 2) the glycine co-agonist binding site, or 3) the site of the ion channel.

The NMDA receptor is composed of multiple protein subunits (Seeburg, P.H., The Molecular Biology of Mammalian Glutamate Receptor Channels, *Trends in Neurosci.,* 16, 359-365 (1993)). The protein subunits fall into two categories: NR2 and NR1. The NR2 subunits contain glutamate binding sites, whereas the NR1 subunits contain the glycine binding sites. Five subunits have been cloned to date, namely NR1 and NR2A, NR2B, NR2C and NR2D. Expression studies indicate the functional receptor is composed of at least one NR1 site and one or more of the NR2 sites. Thus, different subtypes of NMDA receptors can be categorized based on their particular NR2 subunit composition. For example, in the adult mammalian brain, the NR1 and NR2A subunits are widely expressed, forming a subtype of NMDA receptor comprising an NR2A subunit. In contrast, NR2B subunit expression is mostly localized in forebrain regions including cortex, hippocampus and striatum; the NR2C subunit is expressed in the cerebellum; and the NR2D subunit is restricted to the midbrain region. NMDA receptor subtypes of corresponding composition can accordingly respectively be found in forebrain, cerebellum, and midbrain.

Compounds that inhibit NMDA receptor activity by interacting at the glutamate, glycine, or receptor-associated ion channel as described above have little (< 10-fold) selectivity across the different NMDA receptor subtypes. That is, such compounds inhibit NMDA receptors with potencies within a 10-foid range regardless of the subunit combination. However, the subunit composition of the NMDA receptor can confer unique physiology with regard to conductance, kinetics, and affinity for certain agonists. For example, the subunit composition of an NMDA receptor has significant effects on its sensitivity to a group of allosteric modulators which include protons, polyamines, Zn²⁺, and oxidizing/reducing agents (Chenard, B.L. and Menniti, F.S., Antagonists Selective for NMDA Receptors Containing the NR2B Subunit, *Current Pharmaceutical Design*, 19N, 5:381-404)). Receptors comprising the NR2B subunit possess a unique site to which compounds may bind, resulting in specific inhibition this subtype of NMDA receptor as compared to NMDA receptors that do not comprise an NR2B subunit (*Ibid*). This unique site is distinct from the glutamate binding site on the NR2B subunit.

Antagonizing NMDA receptors at the NR2B subunit specific binding site can be used to substantially avoid side effects that have been noted at therapeutic drug levels with other non-specific NMDA receptor antagonists. Both glutamate competitive antagonists and channel blocking agents cause cardiovascular effects and psychotic symptoms in man (Chenard and Menniti, *supra*). In rodents, these types of compounds also cause locomotor hyperactivity and a paradoxical neuronal hyperexcitability manifest as neuronal vacuolization in cingulate and retrospienial cortices (*Id*.). Antagonists at the glycine co-agonist site cause less locomoter activation and do not cause neuronal vacuolization at neuroprotective doses in rodents, however physicochemical problems (for example, problems relating to solubility, brain penetration and protein binding) associated with the quinoxalinedione nucleus typical of such compounds have hindered efforts to bring this class of molecules forward in the clinic (*Id*). NMDA receptor antagonists selective for the NR2B subunit offer a means of inhibition without the side effects and psychochemical difficulties described above.

### NR2B Subunit Selective NMDA Receptor Antagonists

Compounds that inhibit NMDA receptors comprising an NR2B. subunit by specific binding to the NR2B subunit have been demonstrated by measurement of inhibition of NMDA-induced current In Xenopus Oocytes cotransfected with the genes expressing the NR1 and NR2B subunits (Chenard and Menniti, *supra).* Specificity for NR2B can be confirmed by observing reduced inhibition of the NMDA-induced current in Xenopus Oocytes cotransfected with an NR1 subunit and an NR2 subunit other than NR2B.

A number of compounds have been found to act as antagonists that target the NR2B subunits of NMDA receptors that contain them. The first compound identified to display significant affinity for the NR2B subunit was ifenprodil. Ifenprodil is both more potent and efficacious for blockade of ion current through NMDA receptors comprised of NR1/NR2B subunits compared to NR1/NR2A, NR2C, or NR2D subunits.

For example, ifenprodil and related compounds have been demonstrated in animal models of pain perception to produce significant analgesic activity (Bernardi, M., Bertolini, A., Szczawinska, K. And Genedani, S., Blockade of the Polyamine Site of NMDA Receptors Produces Antinociception and Enhances the Effect of Morphine, in Mice, *European Journal of Pharmacology*, 298, 51-55, (1996); Taniguchi, K., Shinjo, K., Mizutani, M., Shimada, K., Ishikawa, T., Menniti, F.S. and Nagahisa, A, Antinociceptive Activity of CP-101,606, an NMDA Receptor NR2B Subunit Antagonist, *British Journal of Pharmacology*, 122, 809-812 (1997)).

United States Patent 5,710,168 (issued January 20, 1998) claims the use of certain compounds of formula I, *infra*, having NR2B subunit selectivity for treating a disease or condition which is susceptible to treatment by blocking of NMDA receptor sites, including traumatic brain injury, spinal cord trauma, pain, psychotic conditions, drug addiction, migraine, hypoglycemia, anxiolytic conditions, urinary incontinence, and ischemic events arising from CNS surgery, open heart surgery or any procedure during which the function of the cardiovascular system is compromised.

United States Serial No. 09/397,891, filed September 17, 1999, pertains to a method of treating acute, chronic and/or neuropathic pain comprising administering an NR2B selective NMDA receptor antagonist, for example a compound of formula I, *infra.*

U.S. 5,710,168 and U.S. Serial No. 09/397,891 are both incorporated by reference herein in their entireties.

### Wind-up-like events and Hyperalgesia and Allodynia

An interplay between spinal neuronal systems, both excitatory and inhibitory, determines the pain messages delivered to higher levels of the central nervous system. Under certain circumstances, the incoming messages may be attenuated or enhanced. The latter state (enhancement) is termed central hypersensitivity, whereby low levels of afferent activity are amplified by spinal pharmacological mechanisms. Inhibitory controls are also subject to alteration, so that opioid sensitivity in different pain states is not fixed. The role of spinal inhibitory systems in the control of central hypersensitivity is important, and allodynia can be produced by pathological or pharmacological interference with spinal GABA or glycine. The resultant touch-evoked responses are NMDA-dependent showing that lifting of inhibitions controlling this receptor in otherwise normal animals causes allodynia (Dickenson, A.H., Spinal Cord Pharmacology of Pain, *British Journal of Anaesthesia,* 1995, 75:193-200).

The capacity for pain transmission in nociceptive systems to change can be induced over very short time periods. Brief activation of spinal C-fibers can result in a marked and prolonged increase in the flexion withdrawal reflex in spinal-decerebrate rats. Repeated constant C-fiber stimuli can induce the phenomenon of wind-up whereby the responses of the deep dorsal hom neurons increase dramatically despite a steady input into the spinal cord. Wind-up needs a certain frequency of stimulation, but when produced can augment responses of dorsal hom neurons by as much as 20 times in amplitude and can prolong responses even after the cessation of the pain-inducing stimuli (i.e., the *event") leading to the wind-up. It is therefore likely that wind-up-like events (events leading to wind-up) underlie central hypersensitivity. In the presence of tissue damage, C-fibers will respond to locally generated chemical stimuli and become hypersensitized to chemical, thermal and mechanical stimuli (Dickenson, supra). Examples of locally generated chemical and physical stimuli that can contribute to wind-up include those associated with Inflammation from tissue damage, for example tissue damage from surgery.

### Neurological Damage Associated with Surgery

Moreover, neurological damage may partlcularty follow certain types of surgeries. For example, both short-term and long-term cognitive deficits have been documented in patients after a coronary artery bypass graft (CABG) (McKhann, G.M., ef a/., Cognitive Outcome After Coronary Artery Bypass: A One-Year Prospective Study, Ann. *Thorac*. *Surg*., 1997 63:510-5). In one study, only 12% of patients showed no decline across eight cognitive domains (verbal memory, visual memory, language, attention, visuoconstruction, psychomotor speed, motor speed, and executive function) (*Ibid*).

### Summary of the Invention

This invention provides for prophylactic use of an NR2B subunit selective NMDA antagonist to inhibit neurological damage following an impairment of glucose and/or oxygen supply to or in the brain. This invention also pertains to a discovery that NR2B subunit selective NMDA antagonists can inhibit central sensitization in a mammal caused by or following the occurrence of a repeated or constant nerve stimulation.

Accordingly, this invention provides a method of inhibiting neurological damage caused by impairment of glucose and/or oxygen to the brain In a mammal, which method comprises administering to the mammal prior to the impairment of glucose and/or oxygen to the brain an amount of an NR2B subunit selective NMDA antagonist, which amount is effective in inhibiting neurological damage.

In one embodiment of the above-recited method of inhibiting neurological damage, the NMDA antagonist is administered to the mammal prior to an event having associated therewith risk of impairment of glucose and/or oxygen supply to the brain, such as an event wherein there exists risk of hypoxia, anoxia, asphyxia, or brain ischemia.

In another embodiment of the above-recited method of inhibiting neurological damage, the mammal to whom the NMDA antagonist is administered is a mammal predisposed to or at risk of brain ischemia, for example stroke.

This invention also provides a method of preventing primary hyperalgesia, secondary hyperalgesia, primary allodynia, secondary allodynia, or other pain caused by central sensitization, in a mammal, which method comprises administering to the mammal, prior to affliction with said pain, an amount of an NR2B subunit selective NMDA antagonist, which amount is effective in preventing said pain.

In one embodiment, the NR2B subunit selective NMDA antagonist in each of the preceding methods is compounds of formula I 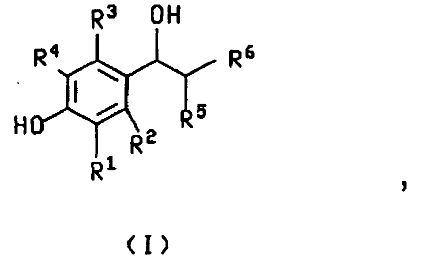 or a pharmaceutically acceptable acid addition salt thereof or an enantiomer thereof, wherein:
(a) R² and R⁵ are taken separately and R¹, R², R³ and R⁴ are each independently hydrogen. (C₁-C₆) alkyl, halo, CF₃, OH or OR⁷ and R⁵ is methyl or ethyl; or
(b) R² and R⁵ are, taken together, 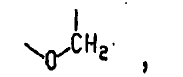 thereby forming a chroman-4-ol ring, and R¹, R³ and R⁴ are each independently hydrogen, (C₁-C₆) alkyl, halo, CF₃, OH or OR⁷;
   R⁶ is 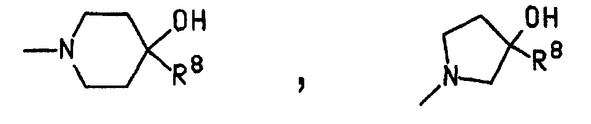 or 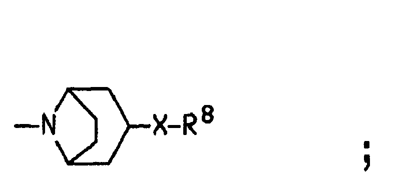 R⁷ is methyl, ethyl, isopropyl or n-propyl;
   R⁸ is phenyl optionally substituted with up to three substituents independently selected from the group consisting of (C₁-C₆) alkyl, halo and CF₃;
   X is O, S or (CH₂)ₙ; and
   n is 0, 1, 2, or 3.
   In another embodiment of each of the preceding methods, the NR2B subunit selective NMDA antagonist is:
   (+)-(1S,2S)-1-(4-hydroxy-phenyl)-2-(4-hydroxy-4-phenylpiperidino)-1-propanol;
   (1S,2S)-1-(4-hydroxy-3-methoxyphenyl)-2-(4-hydroxy-4-phenylpiperidino)-1-propanol;
   (3R,4S)-3-(4-(4-fluorophenyl)-4-hydroxypiperidin-1-yl)-chroman-4,7-diol; or
   (1R*, 2R*)-1-(4-hydroxy-3-methylphenyl)-2-(4-(4-fluorophenyl)-4-hydroxypiperidin-1-yl)-propan-1-ol; or
   an enantiomer of one of the aforementioned compounds; or
   a pharmaceutically acceptable acid addition salt of one of the aforementioned compounds or one of their enantiomers.

### Detailed Description of the Invention

NR2B subunit selective NMDA antagonists that can be used in the methods of the present invention include compounds of formula I 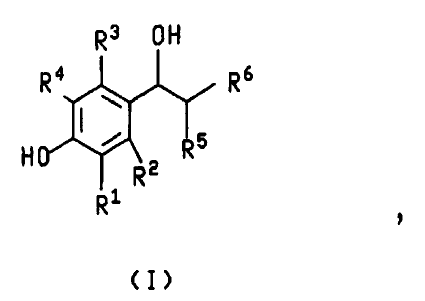 and pharmaceutically acceptable acid addition salt thereof, wherein:
(a) R² and R⁵ are taken separately and R¹, R², R³ and R⁴ are each independently hydrogen, (C₁-C₆) alkyl, halo, CF₃, OH or OR⁷ and R⁵ is methyl or ethyl; or
(b) R² and R⁵ are, taken together, 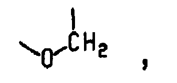 thereby forming a chroman-4-ol ring, and R¹, R³ and R⁴ are each independently hydrogen, (C₁-C₆) alkyl, halo, CF₃, OH or OR⁷;
   R⁸ is 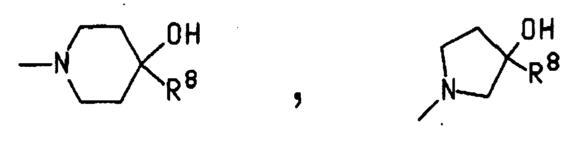 or 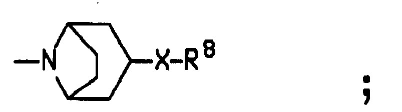 R⁷ is methyl, ethyl, isopropyl or n-propyl;
   R⁸ is phenyl optionally substituted with up to three substituents independently selected from the group consisting of (C₁-C₆) alkyl, halo and CF₃;
   X is O, S or (CH₂)ₙ; and
   n is 0, 1, 2, or 3.

Specific compounds of formula I that can be used are:
(+)-(1S, 2S)-1-(4-hydroxy-phenyl)-2-(4-hydroxy-4-phenylpiperidino)-1-propanol;
(1S, 2S)-1-(4-hydroxy-3-methoxyphenyl)-2-(4-hydroxy-4-phenylpiperidino)-1-propanol;
(3R,4S)-3-(4-(4-fluorophenyl)-4-hydroxypiperidin-1-yl)-chroman-4,7-diol;
(1R*, 2R*)-1-(4-hydroxy-3-methylphenyl)-2-(4-(4-fluorophenyl)-4-hydroxypipendin-1-yl)-propan-1-ol;
enantiomers thereof; and
pharmaceutically-acceptable salts of the above compounds and their enantiomers.

The compounds of formula I can be prepared as follows. The compounds of formula I wherein R² and R⁵ are taken together forming a chroman-4-ol ring, and R¹, R³, and R⁴ are hydrogen, can be prepared by one or more of the synthetic methods described and referred to in United States Patent 5,356,905 (incorporated herein by reference, supra). The compounds of formula I wherein R² and R⁶ are taken separately, and R¹, R², R³ and R⁴ are hydrogen can be prepared by one or more of the synthetic methods described and referred to in United States Patents 5,185,343; 5,272,160; and 5,338,754; all of which are incorporated herein by reference in their entireties. The compounds of formula I can also be prepared by one or more of the synthetic methods described and referred to in United States Patent 6.046,213 (issued April 4, 2000); United States Patents 5,744,483 (issued April 28, 1998) and 6,008,233 (issued December 28,1999); PCT international Application No. PCT/IB95/00398 which designates the United States (filed May 26, 1995) (corresponding to WO 96/37222); and PCT International Application No. PCT/IB95/00380 which designates the United States (filed May 18, 1995) (corresponding to WO 96/06081). These United States Patents and PCT International Applications, and the United States patent application, are also all incorporated by reference herein in their entireties.

A preferred compound, (1S,2S)-1-(4-hydroxyphenyl)-2-(4-hydroxy-4-phenylpipendin-1-yl)-1-propanol ((1S,2S) free base), and its tartrate salt, can be prepared as described in United States Patent 5,272,160, referred to above. The resolution of racemic 1-(4-hydroxyphenyl)-2-(4-hydroxy-4-phonylpiperidin-1-yl)-1-propenol to form the (1S,2S) free base and the corresponding (1 R,2R) enantiomer can be carried out as described In United States Patent 6,008,233 (issued December 28, 1999), referred to above, and as exemplified in Example 1 below.

The anhydrous mesylate of the (1S,2S) free base can be prepared as described in United States Patent 5,272,160, referred to above. The anhydrous mesylate of the (1S,2S) free base, when equilibrated in an 81% relative humidity environment, will convert to the mesylate salt trihydrate of the (1 S,2S) enantiomer.

The mesylate salt trihydrate of (1S,2S)-1-(4-hydroxyphenyl)-2-(4-hydroxy-4-phenylpiperidin-1-yl)-1-propanol can be prepared from the (1S,2S) free base as described in the United States Patent 6,008,233, entitled "(1S,2S)-1-(4-Hydroxyphenyl)-2-(4-Hydroxy-4-Phenylpiperidin-1-yl)-1-Propanol Methanesulfonate Trihydrate", referred to above and incorporated herein by reference in its entirety. In this method, (1S,2S) free base is dissolved in water at 30°C. To this solution is added at least 1 equivalent of methane sulfonic acid and the resulting mixture is warmed to 60-65°C. The warm solution can be filtered to render it particulate free. The solution is concentrated to approximately 40% of the initial volume, cooled below 10°C, isolated by filtration and dried to a water content (measured Karl Fischer titration) of approximately 11.3%. The resulting crystalline mesylate salt trihydrate can be further purified by recrystallization.

Another preferred compound, (3R,4S)-3-[4-(4-fluorophenyl)-4-hydroxy-piperidin-1-yl]-chroman-4,7-diol ((3R,4S) chromanol), can be prepared as described in United States Patent 5,356,905, United States Patent 5,744,483 (issued April 28, 1998), and United States provisional patent application entitled "Process For The Resolution Of Cis-Racemic 7-Benzyloxy-3-[4-(4-Fluorophenyl)-4-Hydroxy-Piperidin-1-yl]-Chromen-4-ol Dibenzoyl-D-Tartrate", all three of which are referred to above. The starting materials and reagents required for the synthesis of the (3R,4S) chromanol are readily available, either commercially, according to synthetic methods disclosed in the literature, or by synthetic methods exempted in the description provided below.

The (3R,4S) chromanol can be prepared by fractional crystallization of the L-proline ester of racemic cis-7-benzyloxy-3-[4-(4-fluorophenyl)-4-hydroxy-piperidin-1-yl]-chroman-4-ol, as described in United States Patent 5,744,483, referred to above. In a preferred method, the resolution method described in United States provisional patent application entitled "Process For The Resolution Of Cis-Racemic 7-Benzyloxy-3-[4-(4-Fluorophenyl)-4-Hydroxy-Piperidin-1-yl]-Chroman-4-ol Dibenzoyl-D-Tartrate", referred to above, and as exemplified in Example 3. In this method, the parent chromanol is prepared by dissolving racemic cis-7-benzyloxy-3-[4-(4-fluorophenyl)-4-hydroxy-piperidin-1-yl]-chroman-4-ol with an equal molar amount of dibenzoyl-D-tartaric acid in boiling aqueous ethanol. Racemic cis-7-benzyloxy-3-[4-(4-fluorophonyl)-4-hydroxy-pipendin-1-yl]-chroman-4-ol is prepared as described in U.S. patent application serial no. 08/189,479, referred to above. The concentration of aqueous ethanol is not critical and may be varied between 75% and 95% ethanol (ETOH). A concentration of 9:1/ETOH:H₂O has been found to be effective and is preferred. A sufficient amount of the aqueous ethanol solvent to dissolve the racemic compound is required. This amount has been found to be about 17ml per gram of racemic compound.

Upon stirring while heating under reflux, the racemic compound dissolves to form a hazy solution which is allowed to cool with stirring whereupon the (+) isomer, (3R,4S)-7-benzyloxy-3-[4-(4-fluorophenyl)-4-hydroxy-piperidin-yl]-chroman-4-ol dibenzoyl-D-tartrate, precipitates and may be collected by filtration and washed with aqueous ethanol. This is the tartrate salt of the (3R,4S) chromanol. The lactate and mandelate salts of the (3R,4S) chromanol are prepared in an analogous manner. This initial product is of about 90% optical purity. If a higher purity is desired, the product may be heated again with aqueous ethanol, cooled and the product collected and washed. Two such treatments were found to yield the (+) isomer of 99.4% optical purity in an overall yield of 74%. This method avoids a reduction step with lithium aluminum hydride and is therefore preferable for bulk operations. This method also can produce a significantly higher yield of the desired product.

The above described (+) isomer can be converted to (3R,4S)-3-[4-(4-fluorophenyl)-4-hydroxy-piperidin-1-yl]-chroman-4,7-diol by standard procedures. For example, treatment with dilute base can be used to free the piperidinyl base and subsequent hydrogeneration removes the 7-benzyl group to yield the (3R,4S) chromanol.

NR2B subunit selective NMDA receptor antagonists useful in the practice of the invention may also be used in the form of a pharmaceutically acceptable salt. The expression "pharmaceuticatlly-acceptable acid addition salts" is intended to include but not be limited to such salts as the hydrochloride, hydrobromide, sulfate, hydrogen sulfate, phosphate, hydrogen phosphate, dihydrogenphosphate, acetate, succinate, citrate, tartrate, lactate, mandelate, methanesulfonate (mosylate) and p-toluenesulfonate (tosylate) salts. The acid addition salts of the compounds of the present invention are readily prepared by reacting the base forms with the appropriate acid. When the salt is of a monobasic acid (e.g., the hydrochloride, the hydrobromide, the p-toluenesulfonate, the acetate), the hydrogen form of a dibasic acid (e.g., the hydrogen sulfate, the succinate) or the dihydrogen form of a tribasic acid (e.g., the dihydrogen phosphate, the citrate), at least one molar equivalent and usually a molar excess of the acid is employed. However when such salts as the sulfate, the hemisuccinate, the hydrogen phosphate or the phosphate are desired, the appropriate and exact chemical equivalents of acid will generally be used. The free base and the acid are usually combined in a co-solvent from which the desired salt precipitates, or can be otherwise isolated by concentration and/or addition of a non-solvent.

Any other compound that is an NR2B subunit selective NMDA receptor antagonist, including its pharmaceutically acceptable salts, can be used in the methods of this invention. NMDA receptor antagonists having NR2B subunit selectivity that may be used according to the present invention are, for example, described in United States Patents 6,046,213; 5,185,343; 5,272,160, 5,338,754; and 5,356,905 (which issued, respectively, on April 4, 2000; February 9, 1993; December 21, 1993; August 16, 1994; and October 18, 1994); United States Patent 6,046,213 (issued April 4, 2000), United States Patents 5,744,483 (issued April 28, 1998) and 6,008,233 (issued December 28, 1999); PCT International Application No. PCT/IB95/00398 (filed May 26, 1995, corresponding to WO 96/37222); and PCT International Application No. PCT/IB95/00380 (filed May 18, 1995, corresponding to WO 96/06081). Other NR2B subunit selective NMDA receptor antagonists that may be used according to the present invention are described in WO 97/32581 (International Publication Date September 12, 1997), WO 98/18793 (International Publication Date May 7, 1998), WO 97/23202 (International Publication Date July 3, 1997), EP 0 824 098 A1 (Date of Publication, February 18, 1998), EP 0846 683 A1 (Date of Publication, June 10, 1998), and DE 19739331 (published November 26, 1998). All of the foregoing patents and published patent applications are incorporated by reference herein in their entireties.

Other compounds that are indicated to bind selectively to NR2B NMDA receptor subunits that may be used according to the present invention are *ifenprodil*, supra, eliprodil (described in United States Patent 4,690,931 (issued September 1, 1987); and compounds described in WO 97/23458 (international Publication Date July 3, 1997), WO 97/23216 (International Publication Date July 3, 1997); WO 97/23215 (International Publication Date July 3, 1997); and WO 97/23214 (International Publication Date July 3, 1997). The preceding U.S. Patent and PCT International Applications are incorporated by reference herein in their entireties.

Compounds that selectively antagonize NMDA receptors comprising an NR2B subunit by specifically binding to the NR2B subunit can be determined by screening compounds for inhibition of NMDA-induced current in recombinant Xenopus Oocytes cotransfected with the NR1A subunit and the NR2B subunit (see, e.g., Monyer, et al., Science, 1992, 256:1217-1221). A compound's activity in inhibiting current in the recombinant cells comprising the NR2B subunit can be compared to its activity inhibiting NMDA-induced current in recombinant Xenopus Oocytes expressing the NR1 subunit and NR2A, NR2C, and NR2D subunits. (See, Chenard and Menniti, *supra).*

One general method that can also generally predict whether or not a compound has NR2B subunit selectivity, for purposes of the present invention, Is a standard competitive binding assay using [³H] radiolabeled racemic CP-101,606 (which contains [³H] (+)-(1S,2S)-1-(4-hydroxy-phenyl)-2-(4-hydroxy-4-phanylpiperidino)-1-propenol; see, for example, U.S. Patent 6,046,213). If a compound has an IC₅₀ of less than about 5µM for inhibition of racemic [³H] CP-101,606 binding to the NR2B subunit, than the compound has NR2B subunit selectivity for purposes of the present invention. An example of such an assay is as follows:

*Example of NR2B subunit binding assay.* Selectivity of compounds for the NR2B-subunit containing NMDA receptor can be defined as an affinity for the racemic [³H] CP-101,606 binding site in forebrain of rats, as described in Chenard and Menniti, *supra.* This affinity is assessed in a radioligand binding assay as described below. Selective compounds are preferably those which displace specific binding of racemic [³H]CP-101,606 from rat forebrain membranes with an IC₅₀ of about ≤ 5 µM.

The binding of racemic [³H] (+)-(1S, 2S)-1-(4-hydroxy-phenyl)-2-(4-hydroxy-4-phenylpiperidino)-1-propanol to rat forebrain membranes is measured as described by Menniti *et al.* (CP-101,606, a potent neuroprotectant selective for forebrain neurons, *European Journal of Pharmacology,* 1997, 331:117-126). Forebrains of adult male CD rats are homogenized in 0.32M sucrose at 4°C. The crude nuclear pellet is removed by centrifugation at 1,000 x g for 10 min., and the supernatant centrifuged at 17,000 x g for 25 min. The resulting pellet is resuspended in 5mM Tris acetate pH 7.4 at 4°C for 10 min. to lyse cellular particles and again centrifuged at 17,000 x g. The resulting pellet is washed twice in Tris acetate, resuspended at 10mg protein/ml and stored at -20°C until use.

For binding assays, membranes are thawed, homogenized, and diluted to 0.5mg protein/ml with 50mM Tris HCl, pH 7.4. Compounds under study are added at various concentrations followed by racemic [³H] CP-1 01,606 (specific activity 42.8 Ci/mmol, 5nM final concentration). Following incubation for 20 min at 30°C in a shaking water bath, samples are filtered onto Whatman GFB glass fiber filters using a MB-48R Cell Harvester (Brandel Research and Development Laboratories, Gaithersburg MD). Filters are washed for 10 s with ice cold Tris HCl buffer and the radioactivity trapped on the filter quantified by liquid scintillation spectroscopy. Nonspecific binding is determined in parallel incubations containing 100µM racemic CP-101,606. Specific binding is defined as total binding minus nonspecific binding.

In one embodiment of the present invention, an NR2B subunit selective NMDA antagonist is furthermore selective for NR2B subunit-containing NMDA receptors over α₁₋adrengergic receptors. For example, although ifenprodll *(supra)* has selectivity for the NR28 subtype of NMDA receptor, this compound is also a well known α₁-adrenergic receptor antagonist. (Carter *et al.* J. Pharmacol. Exp. Ther., 235, 475-482 (1990)). Compounds that antagonize α₁-adrengergic receptors can cause a reduction in blood pressure that can be a complication to therapeutic use. Preferably, the NMDA antagonist has a ratio of NR2B receptor activity to α₁-adrenergic receptor activity of at least about 3:1, more preferably at least about 5:1.

Affinity for the NR2B subunit containing NMDA receptor is measured as the IC₅₀ for displacement of specific binding of racemic [³H] (+)-(1S, 25)-1-(4-hydroxy-phenyl)-2-(4-hydroxy-4-phenylpiperidino)-1-Propanol from rat forebrain membranes (desaibed above). Affinity for the α₁-adrengergic receptor is defined as the IC₅₀ for displacement of specific binding of racemic [³H]prazosin from rat brain membranes, measured as described by Greengrass and Bremner (Binding Characteristics of [³H]prazosin to Rat Brain α-Adrenergic Receptors, *European Journal of Pharmacology,* 55, 323-326, (1979)). A compound with a ratio of ([³H]prazosin/[³H] (+)-(1S, 2S)-1-(4-hydroxy-phenyl)-2-(4-hydroxy-4-phenylpiperidino)-1-propanol) affinity greater than three is considered selective,

Forebrains of adult male Sprague Dawley rats are homogenized in 20 volumes of ice cold 50mM Tris/HCl buffer (pH 7.7). The homogenate is centrifuged at 50,000 X g for 10 min. at 4°C. The pellet is resuspended and centrifuged under identical conditions and the final pellet is resuspended in 80 volumes of 50mM Tris/HCl (pH 8.0) at 4°C.

For binding assays, compounds under study are added at various concentrations to 500 pg membrane protein in 1 ml of 50mM Tris/HCl buffer, followed by [³H]prazosin (Amersham, specific activity 33Ci/mmol, 0.2nM final concentration). Following incubation for 30 min at 25°C in a shaking water bath, samples are filtered onto Whatman GFB glass fiber filters using a MB-48R Cell Harvester (Brandel Research and Development Laboratories, Gaithersburg MD). Filters are washed three times for 10s with ice cold Tris HCl buffer and the radioactivity trapped on the filter quantified by liquid scintillation spectroscopy. Nonspecific binding is determined in parallel incubations containing 100nM prazosin. Specific binding is defined as total binding minus nonspecific binding.

"Inhibiting neurological damage" means a reduction of neurological damage following impairment of glucose and/or oxygen supply to or in the brain compared to the neurological damage that would otherwise have occurred had the NMDA antagonist not been administered.

Neurological damage that is "caused by" impairment of glucose and/or oxygen supply is neurological damage caused at least in part by an insufficiency in the level of glucose and/or oxygen in the brain.

Examples of events having associated therewith risk of brain ischemia include surgeries, especially surgeries pertaining to the lungs, the cardiovascular system (particularly the cerebrovascular system), or the central nervous system. However, any type of surgery carries with it a risk of brain ischemia. One specific example of a type of surgery wherein the risk of ischemic injury is relatively high is a coronary artery bypass graft (CABG). Other examples are cardiac surgery (for example heart surgery), anglography, and angioplasty. Patients undergoing CABG or other surgeries that have associated therewith a high risk of brain ischemia can benefit from an NR2B subunit selective NMDA antagonist.

Other events wherein oxygen supply to the brain may be impaired are events wherein there is a risk of hypoxia, anoxia, or asphyxia. It is thus also beneficial to administer to a mammal, according to the present invention, an NR2B subunit selective NMDA antagonist prior to an event wherein there is a risk of hypoxia, anoxia, or perinatal asphyxia.

Other examples wherein risk of glucose and/or oxygen impairment to or In the brain may be predicted or likely are in patients predisposed to or at risk of brain ischemia, for example stroke. If, for example, a patient has suffered a prior stroke, or has suffered a cardiovascular disease or other condition that impairs the cardiovascular system, that patient may be determined to be predisposed to or at risk of brain ischemia such as stroke. Examples of cardiovascular diseases or other conditions that can impair the cardiovascular system include, but are not limited to, heart-failure, atrial fibrillation, cardiac ischemia, a hypercoagulative state, birth-control pill use, estrogen replacement therapy, poor circulation, atherosclerosis, or congestive heart failure.

In the method of this invention of inhibiting neurological damage resulting from impairment of glucose and/or oxygen supply to or in the brain, the NR2B subunit selective NMDA antagonist is preferably administered prior to the event, for example "surgery", comprising a risk of impairment of glucose and/or oxygen to or in the brain, for example a risk of brain ischemia. Or, as another example, the NR2B subunit selective NMDA antagonist is administered prior to an event wherein there exists a risk of hypoxia, anoxia, or perinatal asphyxia.

This invention also provides a method of preventing primary hyperalgesia, secondary hyperalgesia, primary allodynia, secondary allodynia, or other pain caused by central sensitization, in a mammal, which method comprises administering to the mammal, prior to affliction with said pain, an amount of an NR28 subunit selective NMDA antagonist, which amount is effective in preventing said pain. In one embodiment, the method is for treating hyperalgesia (either primary or secondary), in another embodiment, the method is for preventing allodynia (either primary or secondary), and in another embodiment the invention is for preventing pain caused by central sensitization.

In another embodiment, the NR2B subunit selective NMDA antagonist is administered to the mammal following tissue damage in the mammal. In another embodiment, the NR2B subunit selective NMDA antagonist is administered to the mammal following a "wind-up like event".

"Central sensitization", as used herein, refers to the physiological process in the central nervous system whereby the sensitivity to stimulation is increased due to the occurrence of a repetitive or constant stimulation.

The term "primary", as used herein in "primary hyperalgesia" and "primary allodynia", refers to pain perceived at the site of an injury, stimulation, or tissue damage.

The term "secondary", as used herein in "secondary hyperalgesia" and "secondary allodynia", refers to pain stemming from an injury, stimulation, or tissue damage that is perceived at a site other than the site of the injury, stimulation, or tissue damage, for example at a site adjacent to the site of the injury, stimulation, or tissue damage.

"Hyperalgesia" refers to an increase in a psychometrically measured pain response to a repeated or constant painful stimulation.

"Allodynia" refers to a perception of a non-painful stimulation as painful.

"Wind-up-like event" refers to an occurrence that has the potential to or is likely to bring about central sensitization in a mammal. Generally, a "wind-up-like event", as used herein, is an event which comprises a repeated or constant nerve stimulation (either painful or non-painful). The stimulation can be from an external source, for example a laceration or bullet wound. Or, the stimulation can be initially an external stimulation, which in turn brings about an internal, locally-generated stimulation such as inflammation. Or, the stimulation can be internal, for example tissue inflammation caused by a tumor or a viral infection.

"Tissue damage" includes both direct nerve damage, as well as damage to any tissue (for example, skin or bone) that causes repeated or constant nerve stimuli. Examples of tissue damage are damage from a burn; a laceration; a wound; surgery; a viral infection, for example an HIV infection, a chicken pox infection, a herpes infection, shingles, mumps, or measies; a bone break or fracture; diabetes; arthritis, including rheumatoid arthritis and osteoarthritis, or another musculo-skeletal disorder, cancer; a benign tumor or cyst; a skin disorder or other condition of benign abnormal cell-growth, for example psoriasis; migraine; an implant or a transplant; or a spinal disk injury.

"Prevention", as in "preventing pain", as used herein and unless otherwise indicated, means causing a reduction in the level of pain perceived by a mammal relative to the level that would have been perceived absent administration of the antagonist.

Pain that is "caused by" or "results from" central sensitization is pain in which central sensitization is a contributing factor.

Other examples of pain that can result from a wind-up-like event include, but are not limited to, central pain, stump pain, causalgia, sympathetically maintained pain, phantom limb pain, temporal lobe epilepsy, painful peripheral neuropathy, migraine aura, sensory neural hearing loss, and presbyacusis.

Surgery is a type of event wherein often times there exists a risk of brain ischemia, as discussed above, or risk of inhibition of blood or glucose supply to the brain. Furthermore, central sensitization is a risk in surgery due to incisions and other tissue damage occurring in surgery. Therefore, use of an NR2B subunit selective NMDA receptor antagonist prior to surgery comprises the multiple benefits of inhibiting neurological damage caused by potential brain ischemia and preventing pain caused by potential central sensitization resulting from the surgery. Accordingly, the present invention in one embodiment, comprises administration of an NR2B subunit selective NMDA receptor antagonist prior to a surgery to inhibit neurological damage and/or prevent pain caused by central sensitization.

An effective amount of the NR2B subunit selective NMDA antagonist in inhibiting neurological damage is typically from about 0.02 to 250 mg/kg/day (0.001-12.5 g/day in a typical human weighing 50 kg) in single or divided doses, regardless of route of administration. A more preferred dosage range is from about 0.15 mg/kg/day to about 250 mg/kg/day.

An effective amount of the NR2B subunit selective NMDA antagonist in preventing hyperalgesia or allodynia in a mammal, is about 0.02mg to about 10 mg/kg/day (1 to 500mg/day in a typical human weighing 50kg.

Of course, depending upon the specific compound and circumstances (for example, the presence or absence of a predisposition to neurological damage, even doses outside the aforementioned ranges may be in order. A suitable dose given specific circumstances can be determined by a physician or other health-care professional of ordinary skill.

The NR2B selective NMDA receptor antagonist useful in the method of the present invention is generally administered in the form of a pharmaceutical composition comprising one or more NR2B selective NMDA receptor antagonists together with a pharmaceutically acceptable carrier or diluent. It may also be beneficial for the methods of the present invention to co-administer, either in the same composition or in separate compositions, the NR2B selective NMDA receptor antagonist(s) with one or more other substances, for example anesthetics, analgesics and/or antianxiolytics.

The compositions described herein useful in the present invention are generally formulated in a conventional manner utilizing solid or liquid vehicles or diluents as appropriate to the mode of administration. For purposes of oral administration, tablets containing excipients such as sodium citrate, calcium carbonate and dicalcium phosphate may be employed along with various disintegrants such as starch and preferably potato or tapioca starch, alginic acid and certain complex silicates, together with binding agents such as polyvinylpyrrolidone, sucrose, gelatin and acacia. Additionally, lubricating agents such as, but not limited to, magnesium stearate, sodium lauryl sulfate and talc are often very useful for tableting purposes. Solid compositions of a similar type may also be employed as fillers in soft elastic and hard-filled gelatin capsules; preferred materials in this connection also include, by way of example and not of limitation, lactose or milk sugar as well as high molecular weight polyethylene glycols. When aqueous suspensions and/or elixirs are desired for oral administration, the essential active ingredient may be combined with various sweetening or flavoring agents, coloring matter or dyes and, if so desired, emulsifying and/or suspending agents, together with diluents such as water, ethanol, propylene glycol, glycerin and various like combinations thereof.

The present invention is illustrated by the following examples, but is not limited to the details thereof.

All nonaqueous reactions were run under nitrogen for convenience and generally to maximize yields. All solvents/diluents were dried according to standard published procedures or purchased in a predried form. All reactions were stirred either magnetically or mechanically. NMR spectra are recorded at 300 MHz and are reported in ppm. The NMR solvent was CDCl₃ unless otherwise specified. IR spectra are reported In cm⁻¹, generally specifying only strong signals.

### EXAMPLE 1

### Enantiomeric (1S, 2S)- and (1R, 2R)-1-(4-Hydroxyphenyl)-2-(4-hydroxy-4-phenylpiperidin-1-yl)-1-propanol

(+)-Tartaric acid (300mg, 2mmol) was dissolved in 30mL warm methanol. Racemic 1S*, 2S*-1-(4-hydroxyphenyl)-2-(4-hydroxy-4-phenylpiperidin-1-yl)-1-propanol (655mg, 2mmol) was added all at once. With stirring and gentle warming a colorless homogeneous solution was obtained. Upon standing at ambient temperature 24 hours, 319mg (66%) of a fluffy white precipitate was obtained. This product was recrystallized from methanol to give 263mg of the (+)-tartrate salt of levorotatory title product as a white solid; mp 206.5-207.5°C; [alpha]_{D} = -36.2°. This salt (115 mg) was added to 50 mL of saturated NaHCO₃. Ethyl acetate (5mL) was added and the mixture was vigorously stirred 30 minutes. The aqueous phase was repeatedly extracted with ethyl acetate. The organic layers were combined and washed with brine, dried over calcium sulfate, and concentrated. The tan residue was recrystallized from ethyl acetate-hexane to give 32mg (39%) of white, levorotatory title product; mp 203-204°C; [alpha]_{D} = -58.4°. Anal. Calc'd. for C₂₀H₂₅NO₃: C, 73.37; H, 7.70; N, 4.28. Found: C, 72.61; H, 7.45; N, 4.21.

The filtrate from the (+)-tartrate salt preparation above was treated with 100mL saturated aqueous NaHCO₃ and extracted well with ethyl acetate. The combined organic extracts were washed with brine, dried over calcium sulfate and concentrated to give 380 mg of recovered starting material (partially resolved). This material was treated with (-)-tartaric acid (174 mg) in 30mL of methanol as above. After standing for 24 hours, filtration gave 320 mg (66%) of product which was further recrystallized from methanol to produce 239 mg of the (-)-tartrate salt of dextrorotatory title product; mp 206.5-207.5°C [alpha]_{D} = +33.9°. The latter was converted to dextrorotatory title product in the manner above in 49% yield; mp 204-205°C; [alpha]_{D} = +56.9°. Anal. Found: C, 72.94; H. 7.64; N, 4.24.

### EXAMPLE 2

### (1S, 2S)-1-(4-hydroxyphenyl)-2-(4-hydroxy-4-phenylpiperidin-yl)-1-propanol methanesulfonate trihydrate

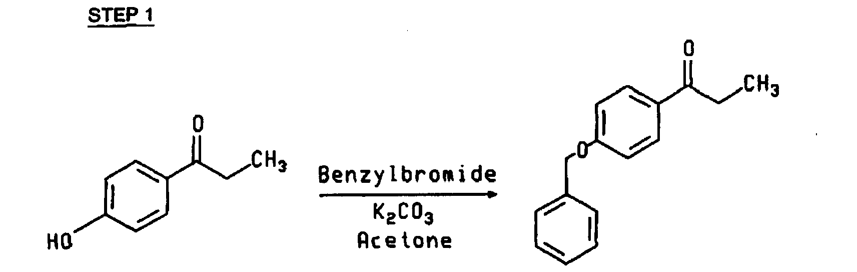

A 50 gallon glass lined reactor was charged with 17.1 gallons of acetone, 8.65 kilograms (kg) (57.7mol) of 4'-hydroxypropiophenone, 9.95kg (72.0mol) of potassium carbonate and 6.8 liters (I) (57.7mol) of benzylbromide. The mixture was heated to reflux (56°C) for 20 hours. Analysis of thin layer chromatography (TLC) revealed that the reaction was essentially complete. The suspension was atmospherically concentrated to a volume of 10 gallons and 17.1 gallons of water were charged. The suspension was granulated at 25°C for 1 hour. The product was filtered on a 30" Lapp and washed with 4.6 gallons of water followed by a mixture of 6.9 gallons of hexane and 2.3 gallons of isopropanol. After vacuum drying at 45°C, this yielded 13.35 kg (96.4%) of the above-depicted product.

A second run was carried out with 9.8kg (65.25mol) of 4'-hydroxypropiophenone using the procedure described above. After drying 15.1 kg (96.3%) of the above-depicted product was obtained.

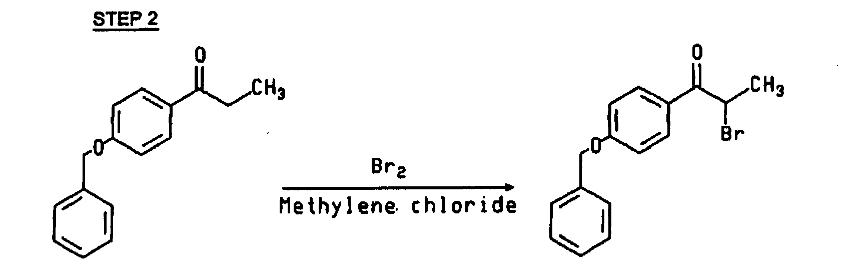

Under a nitrogen atmosphere, a 100 gallon glass lined reactor was charged with 75 gallons of methylene chloride and 28.2kg (117.5mol) of the product from step 1. The solution was stirred five minutes and then 18.8kg of bromine was charged. The reaction was stirred for 0.5 hours at 22°C. Analysis of TLC revealed that the reaction was essentially complete. To the solution was charged 37 gallons of water and the mixture was stirred for 15 minutes. The methylene chloride was separated and washed with 18.5 gallons of saturated aqueous sodium bicarbonate. The methylene chloride was separated, atmospherically concentrated to a volume of 40 gallons and 60 gallons of isopropanol was charged. The concentration was continued until a pot temperature of 80°C and final volume of 40 gallons were obtained. The suspension was cooled to 20°C and granulated for 18 hours. The product was filtered on a 30" Lapp and washed with 10 gallons of isopropanol. After vacuum drying at 45°C, this yielded 29.1 kg (77.6%) of the above-depicted product.

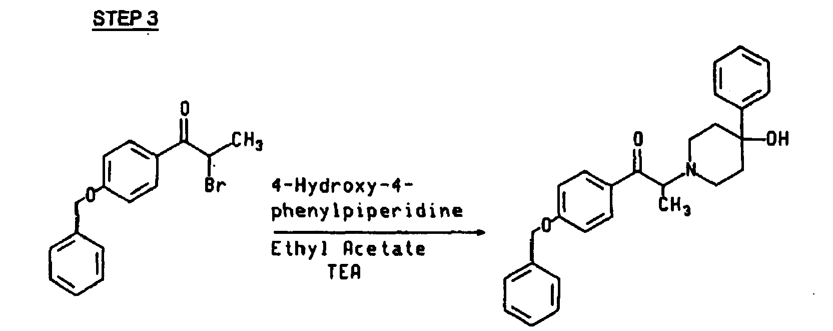

Under a nitrogen atmosphere, a 20 gallon glass lined reactor was charged with 4.90kg (15.3mol) of the product from step 2, 7.0 gallons of ethyl acetate, 2.70kg (15.3mol) of 4-hydroxy-4-phenylpiperidine and 1.54 kg of triethylamine (15.3mol). The solution was heated to reflux (17°C) for 18 hours. The resulting suspension was cooled to 20°C. Analysis by TLC revealed that the reaction was essentially complete. The byproduct (triethylamine hydrobromide salt) was filtered on a 30" Lapp and washed with 4 gallons of ethyl acetate. The filtrate was concentrated under vacuum to a volume of 17 liters. The concentrate was charged to 48 liters of hexane and the resulting suspension granulated for 2 hours at 20°C. The product was filtered on a 30" Lapp and washed with 4 gallons of hexane. After vacuum drying at 50°C, this yielded 4.9kg (77%) of the above-depicted product.

A second run was carried out with 3.6kg (11.3mol) of the product from step 2 using the procedure described above. After drying 4.1 kg (87%) of the above-depicted product was obtained.

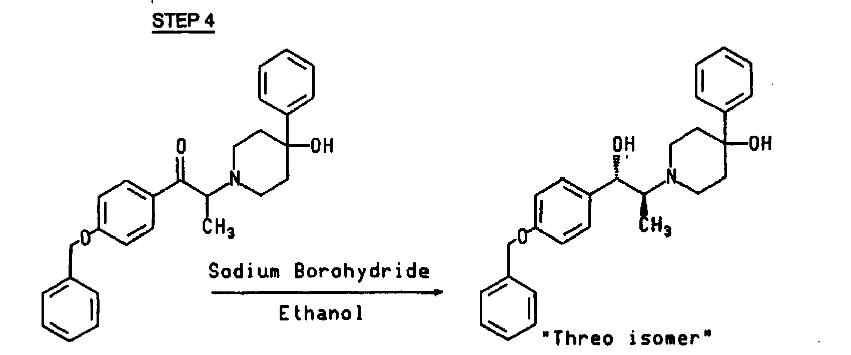

Under a nitrogen atmosphere, a 100 gallon glass lined reactor was charged with 87.0 gallons of 2B ethanol and 1.7kg (45.2mol) of sodium borohydride. The resulting solution was stirred at 25°C and 9.4kg (22.6mol) of the product from step 3 was charged. The suspension was stirred for 18 hours at 25-30°C. Analysis by TLC revealed that the reaction was essentially complete to the desired threo diastereoisomer. To the suspension was charged 7.8 liters of water. The suspension was concentrated under vacuum to a volume of 40 gallons. After granulating for 1 hour, the product was filtered on a 30" Lapp and washed with 2 gallons of 2B ethanol. The wet product, 9.4 gallons of 2B-ethanol and 8.7 gallons of water were charged to a 100 gallon glass lined reactor. The suspension was stirred at reflux (78°C) for 16 hours. The suspension was cooled to 25°C, filtered on 30" Lapp and washed with 7 gallons of water followed by 4 gallons of 2B ethanol. After air drying at 50°C, this yielded 8.2kg (86.5%) of the above-depicted product. This material was recrystallized in the following manner.

A 100 gallon glass lined reactor was charged with 7.9kg (18.9mol) of the product from step 3, 20 gallons of 2B ethanol and 4 gallons of acetone. The suspension was heated to 70°C producing a solution. The solution was concentrated atmospherically to a volume of 15 gallons. The suspension was cooled to 25°C and granulated for 1 hour. The product was filtered on a 30" Lapp. The wet product and 11.7 gallons of 2B ethanol was charged to a 100 gallon glass lined reactor. The suspension was heated to reflux (78°C) for 18 hours. The suspension was cooled to 25°C, filtered on a 30" Lapp and washed with 2 gallons of 2B ethanol. After air drying at 50°C this yielded 5.6kg (70.6%) of the above-depicted product.

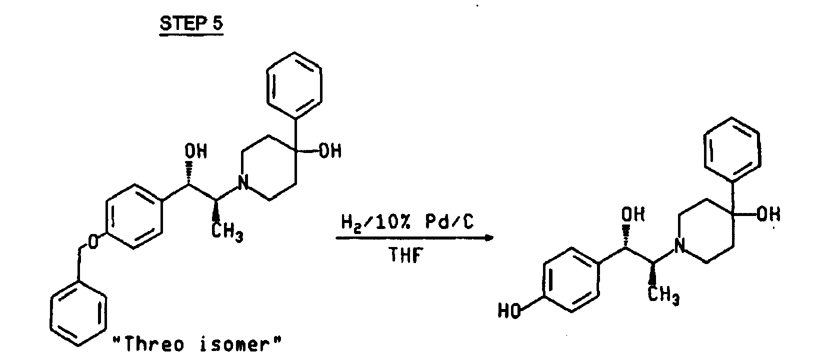

Under a nitrogen atmosphere, a 50 gallon glass lined reactor was charged with 825g of 10% palladium on carbon (50% water wet), 5.5kg (13.2mol) of the product from step 4 and 15.5 gallons of tetrahydrofuran (THF). The mixture was hydrogenated between 40-50°C for 2 hours. At this time, analysis by TLC revealed that the reduction was essentially complete. The reaction was filtered through a 14" sparkler precoated with Celite and washed with 8 gallons of THF. The filtrate was transferred to a clean 100 gallon glass lined reactor, vacuum concentrated to a volume of 7 gallons and 21 gallons of ethyl acetate were charged. The suspension was atmospherically concentrated to a volume of 10 gallons and a pot temperature of 72°C. The suspension was cooled to 10°C, filtered on a 30" Lapp and washed with 2 gallons of ethyl acetate. After air drying at 55°C this yielded a 3.9kg (90%) of the above-depicted product (i.e., the free base).

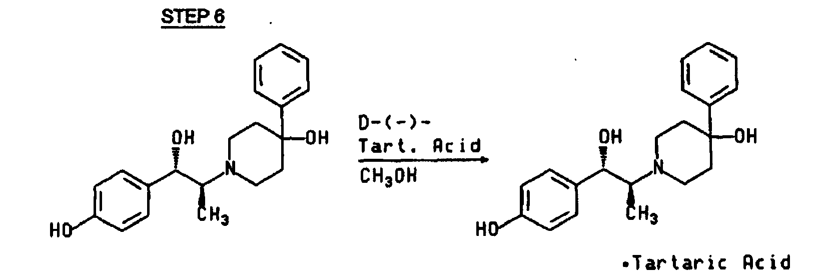

A 100 gallon glass lined reactor was charged with 20 gallons of methanol and 3.7kg (11.4mol) of the product from step 5 (i.e., the free base). The suspension was heated to 60°C and 1.7kg (11.4mol) of D-(-)-tartaric acid were charged. The resulting solution was heated to reflux (65°C) for 3 hours after which a suspension formed. The suspension was cooled to 35°C, filtered on a 30" Lapp and washed with 1 gallon of methanol. The wet solids were charged to a 100 gallon glass lined reactor with 10 gallons of methanol. The suspension was stirred for 18 hours at 25°C. The suspension was filtered on a 30" Lapp and washed with 2 gallons of methanol. After air drying at 50°C this yielded 2.7kg (101%) of the above-depicted product (i.e., the tartaric acid salt of the free base (R-(+)-enantiomr)). This material was purified in the following manner:

A 100 gallon glass lined reactor was charged with 10.6 gallons of methanol and 2.67kg (5.6mol) of the above tartaric acid salt. The suspension was heated to reflux (80°C) for 18 hours. The suspension was cooled to 30°C, filtered on a 30" Lapp and washed with 4 gallons of methanol. After air drying at 50°C, this yielded 2.05kg (76.7%) of the above-depicted product (i.e., the tartaric acid salt of the free base).

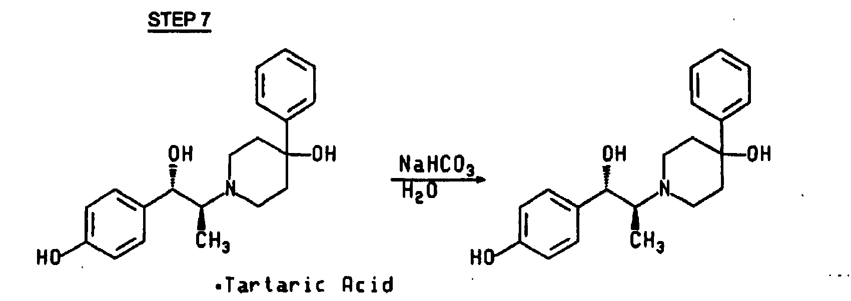

A 55 liter nalgene tub was charged with 30 liters of water and 1056 g (12.6 mol) of sodium bicarbonate at 20°C. To the resulting solution was charged 2.0kg (4.2mol) of the product from step 6 (i.e., the tartaric acid salt of the free base). The suspension was stirred for 4 hours during which a great deal foaming occurred. After the foaming ceased, the suspension was filtered on a 32cm funnel and washed with 1 gallon of water. After air drying at 50°C, this yielded 1.28kg (93.5%) of the above-depicted product (i.e., the free base).

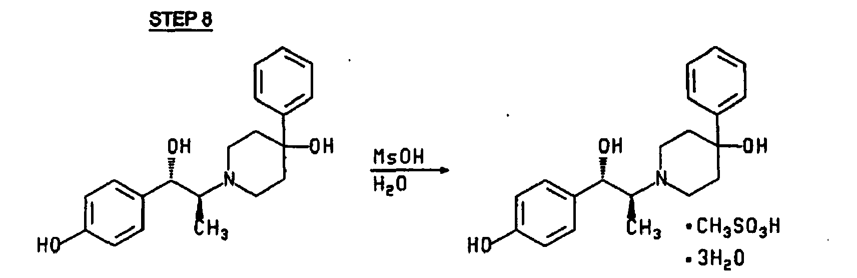

A 22 liter flask was charged with 1277g (3.9mol) of product from step 7 and 14 liters of water. The suspension was warmed to 30°C and 375g (3.9mol) of methane sulfonic acid were charged. The resulting solution was warmed to 60°C, clarified by filtering through diatomaceous earth (Celite™) and washed with 2 liters of water. The speck-free filtrate was concentrated under vacuum to a volume of 6 liters. The suspension was cooled to 0-5°C and granulated for 1 hour. The product was filtered on an 18" filter funnel and washed with 635ml of speck-free water. After air drying at 25°C for 18 hours, this yielded 1646 g (88%) of the above-depicted product (i.e., the mesylate salt trihydrate).

### EXAMPLE 3

### (1R*,2R*)-1-(4-hydroxy-3-methylphenyl)-2-(4-(4-fluorophenyl)-4-hydroxypiperidin-1-yl)-propan-1-ol-mesylate

A mixture of 3-methyl-4-triisopropylsilyloxy-α-bromopropiophenone (9.17g, 22.97mmol), 4-(4-fluorophenyl)-4-hydroxypiperidine (6.73g, 34.45mmol) and triethylamine (8.0mL, 57.43mmol) in in ethanol (180mL) was refluxed for 6 hours. The solvent was removed at reduced pressure and the residue was partitioned between ethyl acetate and water. The phases were separated and the organic layer was washed with brine, dried over calcium sulfate and concentrated. The residue was flash chromatographed on silica gel (3x3.5 inches packed in hexane) with elution proceeding as follows: 10% ethyl acetate/hexane (1000mL), nil; 20% ethyl acetate/hexane (700mL), nil; 20% ethyl acetate/hexane (1300mL) and 25% ethyl acetate/hexane (600mL), 7.66g (65%) of 1-(3-methyl-4-trilsopropylsilyloxyphenyl)-2-(4-(4-fluorophenyl)-4-hydroxypiperidin-1-yl)-propan-1-one as a yellow foam which was suitable for use without further purification. A sample recrystallization from ethyl acetate/hexane as white crystals had: m.p. 78-82°C.

A mixture of sodium borohydride (0.564g, 14.92mmol) and ethanol (60mL) was stirred 10 minutes and then 1-(3-methyl-4-triisopropyphenyl)-2-(4-(4-fluorophenyl)-4-hydroxyplperidin-1-yl)-propan-1-one (7.66g, 14.92mmol in 10mL of ethanol) was added with two 30mL ethanol rinses. The reaction mixture was stirred at ambient temperature overnight. The white solid that precipitated was collected by filtration and dried to yield 5.72g (74%) of (1R*, 2R*)-1-(3-methyl-4-triisopropylsilyloxyphenyl)-2-(4-(4-fluorophenyl)-4-hydroxypiperidin-1-yl)-propan-1-ol, which was suitable for use without further purification and had: m.p. 188-189°C.

The product of the above reaction (5.72g, 11.1mmol) was dissolved in tetrahydrofuran (150mL) and tetrabtitytammonium fluoride (12.21mL, 12.21mmol, 1M tetrahydrofuran solution) was added. The reaction was stirred 1 hour at ambient temperature and then concentrated. The residue was partitioned between ethyl acetate and water and the two phases were separated. The organic layer was slurried with methylene chloride. The white solid that precipitated was collected by filtration and dried to afford 3.41g (85%) of (1R*, 2R*)-1-(4-hydroxy-3-methylphenyl)-2-(4-(4-fluorophenyl)-4-hydroxyperidin-1-yl)-propan-1-ol. A sample (0.16g, 0.441mmol) was converted to the corresponding mesylate salt. The salt was slurried in methanol (8mL) and methanesulfonic acid (0.029mL, 0.45mmol) was added. The mixture was filtered and concentrated. The mixture was then recrystallized from ethanol to give 0.152g (58%) of the mesylate salt which had: m.p. 215-216°C. Analysis calculated for C₂₁H₂₅FNO₃•CH₄SO₃: C, 58.01; H, 6.64, N, 3.07. Found: C, 57.99; H, 6.72: N, 3.17.

### EXAMPLE 4

### 1R, 2R 1-(4-hydroxy-3-methoxyphenyl)-2-(4-hydroxy-4-phenyl-peridin-1-yl)-propan-1-ol and

### 1S, 2S 1-(4-hydroxy-3-methoxyphenyl)-2-(4-hydroxy-4-phenyl-piperidin-1-yl)-propan-1-ol

A mixture of 2-bromo-1-(2,2-diphenyl-benzo(1,3)dioxol-5-yl)-propan-1-one (2.00g, 4.89mmol), 4-hydroxy-4-phenylpiperldine (0.9g. 5.08mmol) and triethylamine (1.40mL, 10.04mmol) in ethanol (50mL) was refluxed overnight. The solvent was removed at reduced pressure and the residue was partitioned between ether and water. The phases were separated and the organic layer was washed with brine, dried over magnesium sulfate and concentrated. The residue was flash chromatographed on silica gel (2x5 inches packed with hexane) with elution proceeding as follows: 20% ethyl acetate/hexane (500mL), unweighed forerun; 50% ethyl acetate/hexane (500mL), 1.76g (71%) of 1-(2,2)-diphenyl-benzo(1,3)dioxol-5-yl)-2-(4-hydroxy-4-phenylpipendin-1-yl)-propan-1-one as light tan foam which was suitable for use without further purification and had: NMR δ 7.81 (dd, J=1.7, 8.3Hz, 1H), 7.70 (d, J=1.6Hz, 1H), 7.64-7.13 (m, 15H), 6.92 (d, J=8.2Hz, 1H), 4.07 (q, J=7.0Hz, 1H), 3.39-3.27 (m, 1H), 2.94-2.59 (m, #H), 2.30-2.04 (m, 2H), 1.74 (br t, J=13.2Hz, 2H), 1.30 (d, J=6.8Hz, 3H).

A mixture of sodium borohydride (0.15g, 3.97mmol) and ethanol (5mL) was stirred 10 minutes and then 1-(2,2-diphenyl-benzo(1,3)dioxol-5-yl)-2-(4-hydroxy-4-phenylpiperidin-1-yl)-propan-1-one (1.70g, 3.36mmol in 20 mL of ethanol) was added. The reaction was stirred at ambient temperature over the weekend. The white precipitate was collected, rinsed with ethanol and ether and air dried to afford 1.35g of crude product. The product was recrystallized from ethanol/ethyl acetate/methylene chloride to give 1.05g (61%) of 1R*, 2R*)-1-(2.2-diphenyl-benzo(1,3)dioxol-5-yl)-2-(4-hydroxy-4-phenylpiperidin-1-yl)-propan-1-ol which had: mp 224-224.5°C. Analysis calculated for C₃₃H₃₃NO₄: C, 78.08; H, 6.55; N, 2.76. Found: C, 78.16; H, 6.46; N, 2.72.

A mixture of the product of the above reaction (1.00g, 1.97mmol) and 10% palladium on carbon (0.175g) in methanol (5OmL) and acetic acid (1.0mL) was hydrogenated at 50psi (initial pressure) for 5 hours at ambient temperature. Additional catalyst (0.18g) was added and the hydrogenation was continued overnight. The reaction was fdtered through diatomaceous earth and the filter pad was rinsed with methanol. The filtrate was concentrated and the residue was partitioned between ethyl acetate and saturated aqueous bicarbonate and stirred vigorously for 1 hour. The phases were separated and the aqueous layer was extracted with ethyl acetate (2x). The combined organic layer was washed with water and brine, dried over magnesium sulfate and concentrated. The residue was flash chromatographed on silica gel (1x4 inches) with elution proceeding as follows: 20% ethyl acetate/hexane (500mL), nil; 10% methanol/ethyl acetate (250mL), 20% methanol/ethyl acetate (250mL), and 50% methanol/ethyl acetate, 0.51g (75%) of a light yellow-green solid. The solid was recrystallized from ethanol to afford (1R*, 2R*)-1-(3,4-dihydroxyphenyl)-2-(4-hydroxy-4-phenyl-piperidin-1-yl)-propan-1-ol as a white solid which had: mp 167-168°C. Analysis calculated for C₂₀H₂₅NO₄·0.5 C₂H₆O: C, 68.83; H, 7.70; N, 3.82. Found: C, 68.78; H, 8.05; N, 3.70.

The racemic product was dissolved in ethanol and separated into enantiomers by HPLC using the following chromatographic conditions: Column, Chiralcel OD; mobile phase, 25% ethanol/75% hexane; temperature, ambient (approximately 22°C); detection, UV at 215nM. Under these conditions, 1R, 2R 1-(4-hydroxy-3-methoxyphenyl)-2-(4-hydroxy-4-phenyl-piperidln-1-yl) propan-1-ol eluted with a retention time of approximately 9.12minuntes and 1S, 2S 1-(4-hydroxy-3-methoxyphonyl)-2-(4-hydroxy-4-phonyl-piperidin-1-yl) propan-1-ol eluted with a retention time of approximately 16.26 minutes.

### EXAMPLE 5

### (3R*, 4S*)-3-(4-(4-fluorophenyl)-4-hydroxypiperidin-1-yl)-chroman-4,7-diol

A mixture of 7-benzyloxy-3,3-dibromochromanone (54.7g, 133mmol), 4-(4-fluorophonyl)-4-hydroxypiperidine (52.0g, 266mmol), and triethylamine (38mL, 270mmol) in acetonitrile (2.5L) was stirred 16 hours at ambient temperature. A yellow precipitate formed and was collected, washed well with water and ether, and air dried. The yield of 7-benzyloxy-3-{4-(4-fluorophenyl)-4-hydroxy-pipridine-1-yl}-chromenone was 55.4g (93%) which was suitable for use without further purification. A sample recrystallized from ethanol/tetrahydrofuran had mp 220-221°C: NMR DMSO_{∂σ} δ7.99 (d, J=9Hz, 2H), 7.56-7.40 (m, 8H), 7.18-7.08 (m, 4H), 5.25 (s, 2H), 5.06 (s, 1H), 3.60 (br s, 1H), 3.55-3.35 (m, 1H, partially obscured by water from the NMR solvent), 3.10-2.95 (m, 2H), 2.15-2.00 (m, 2H), 1.71 (br t, J=13.7Hz, 2H).

Analysis calculated for C₂₇H₂₄FNO₄: C, 72.80; H, 5.43; N, 3.13. Found: C, 72.83; H, 5.82; N, 2.82.

To a slurry of 7-benzyloxy-3-[4-(4-fluorophenyl)-4-hydroxy-piperidine-1-yl]-chromenone (8.24g, 18.5mmol) in ethanol (400mL) and tetrahydrofuran (600mL) was added sodium borohydride (7.0g, 185mmol). The mixture was stirred overnight. Additional sodium borohydride (7.0g) was added and the reaction mixture was stirred for 3 days. Water was added and the solvent was removed at reduced pressure at 45°C. The solids which formed were collected and washed well with water and then ether. The solid was further dried in vacuo overnight to give 5.01g, 60% of 3R* 4S* 7-benzyloxy-3-[4-(4-fluorophenyl)-4-hydroxy-piperidin-1-yl]-chroman-4-ol which was suitable for use without further purification. A sample recrystallized from ethyl acetate/chloroform had mp. 184-195°C; NMR δ7.56-7.30 (m, 8H), 7.06 (long range coupled t, J=8.7Hz, 2H) 6.63 (dd, J=2.4, 8.5Hz, 1H), 6.47 (d, J=2.4Hz, 1H), 5.04 (s, 2H), 4.77 (d, J=4.5Hz, 1 H), 4.37 (dd, J=3.5, 10.4Hz, 1H), 4.13 (t, J=10.4Hz, 1H), 3.82 (brs, 1 H), 3.11 (br d, J=11.2Hz, 1H), 2.92-2.71 (m, 4H), 2.21-2.06(m, 2H), 1.87-1.73 (m, 2H), 1.54 (s, 1H).

Analysis calculated for C₂₇H₂₈FNO₄: C, 72.14; H, 628; N, 3.12. Found C, 72.15; H, 6.21; N, 3.12.

A mixture of 3R* 4S* 7-benzyloxy-3-[4-(4-fluorophenyl)-4-hydroxy-piperidin-1-yl]-chroman-4-ol (0.80g, 1.78mmol), 10% palladium on carbon (0.16g), methanol (40mL), and acetic acid (0.8mL) was hydrogenated for 8 hours with a starting pressure of 48.5psi. The reaction was filtered through celite and the filtrate was concentrated. The residue was stirred vigorously with ether and saturaturated sodium bicarbonate for 1 hour. The solid was washed with water and ether and dried in vacuo. Recrystallization from ethanol yielded 0.35g (54%) of 3R* 4S* 3-[4-(4-fluorophenyl)-4-hydroxy-piperidin-1-yl]-chroman-4,7-diol as a white solid which had mp 159-160°C; NMR DMSO_{∂σ} δ7.55-7.47 (m, 2H), 7.11 (t, J=9Hz. 2H), 7.02 (d, J=8.4Hz, 1H)k, 6.32 (dd, J=2.3, 8.3Hz, 1H), 6.15 (d, J=2.3Hz 1H), 5.10-4.50 (br m with s at 4.63, 3H), 4.23 (dd, J=2.8, 10.3Hz, 1H), 4.04 (t, J=10.5Hz. 1H), 2.99 (br d, J=10.8Hz, 1H), 2.86 (br d, J=10.7Hz, 1 H), 2.73-2.50 (m, 3H), 2.08-1.90 (m, 2H), 1.58 (br d, J=13Hz, 2H).

Analysis calculated for C₂₀H₂₂FNO₄•0.25H₂O; C, 66.01; H, 6.23; N, 3.85. Found: C, 66.22; H, 6.58; N. 3.46.

### EXAMPLE 6

### Pre-surgery Administration of CP-101,606

Using the paw incision model for surgical pain (see, e.g., M.J. Field, et al., *J. Pharmacol. Exp. Ther.* 282:1242-1246 (1997); M.I. Gonzalez, et al., *Eur. J. Pharmacol.* 344:115-120 (1998); or M.J. Field, et al., *PAIN* 80: 383-389 (1999)), CP-101,606 dose-dependently blocked both static and dynamic subtypes of allodynia.

Basically, in this animal model, an incision is made in the hind paw of the rats, which induces a reproducible and quantifiable mechanical hyperalgesia in the rats lasting several days. Rats are anesthetized, and the plantar surface of the right hind paw of each rat is incised through the skin and fascia. The wound is then closed, for example with sutures, and allowed to heal. The rats are placed into wire mesh bottom cages, allowing access to the underside of their paws. After habituation, tactile allodynia (or "static allodynia*) can be evaluated by touching the right hind paw plantar surface with von Frey hairs in ascending order of force (measured in grams) until a paw-withdrawal response (paw withdrawal threshold or "PWT") is elicited. Dynamic allodynia can be evaluated by stoking the plantar surface of the right hind paw and measuring (in seconds) the amount of time it takes for a rat to withdraw its paw (paw withdrawal latency or "PWL").

In the pre-surgery administration assay, a PWT and PWL baseline is measured prior to drug administration and incision. The rats are then given drug (CP-101,606, at separate doses of 3, 10 or 30 mg/kg s.c.) or are given pregabalin (30 mg/kg s.c., as a positive control)), or vehicle (s.c.). The right hind paw each rat is then incised as described above. Measurements are then taken periodically, as described above.

CP-101,606 dose-dependently blocked both static and dynamic subtypes of allodynia, with respective MEDs (minimum effective dosages) of 10mg/kg. CP-101,606 produced a full blockade of static allodynia and a near full blockade of the dynamic subtype. The dose of 30mg/kg had a peak effect at two hours for both static and dynamic allodynia. The antiallodynic action of CP-101,606 lasted for at least 46 hours following pre-surgical administration, and a preventive effect was seen as late as 72 hours post surgery. As late as 72 hours, the PWT was higher and the PWL longer in rats treated pre-surgically with 10 and 30 mg/kg CP-101,606 (and pregabalin) compared to the PWT and PWL for rats who had been given vehicle prior to surgery. These data demonstrate that an NR2B subtype selective NMDA receptor antagonist is effective in preventing pain caused by central sensitization, for example allodynia resulting from a surgical wound.

### EXAMPLE 7

### Post-surgery Administration of CP-101,606

CP-101,606 was also tested post-surgically using the paw incision model, basically as described above. However, in this assay, rats are administered CP-101,606 (3, 10, or 30 mg/kg, s.c.), pregabalin (30 mg/kg, s.c.), or vehicle (s.c.) post-incision. A baseline is measured before incision and after incision, prior to drug. CP-101,606 dose-dependently diminished both static and dynamic subtypes of allodynia, with respective MEDs of 10 and 30 mg/kg. CP-101,606 produced a full reversal of static allodynia and a partial (approximately 40%) reversal of the dynamic subtype. The dose of 30 mg/kg had a peak effect at one hour and two hours respectively for static and dynamic allodynia which lasted for three hours and two hours respectively. These data show that NR2B subtype selective NMDA receptor antagonists can also be used to reduce pain.

## Claims

1. Use of an NR2B subunit selective NMDA antagonist in the manufacture of a medicament for preventing primary hyperplasia, secondary hyperplasia, primary allodynia, secondary allodynia, or other pain caused by central sensitization, in a mammal, **characterised in that** the medicament is administered prior to affliction with said pain.

2. Use according to claim 1, wherein the medicament is administered following tissue damage.

3. Use according to claim 2, wherein the tissue damage is from a burn; a cut; a wound; surgery; a viral infection, for example an HIV infection, a chicken pox infection, a herpes infection, shingles, mumps, or measles; a bone break or fracture; diabetes; arthritis, including rheumatoid arthritis and osteoarthritis, or another musculo-skeletal disorder; cancer; a benign tumour or cyst; a skin disorder or other condition of benign abnormal cell-growth, for example psoriasis; migraine, an implant or a transplant; or a spinal disk injury.

4. Use according to claim 1, wherein the pain caused by central sensitization is central pain, stump pain, causalgia, sympathetically maintained pain, phantom limb pain, temporal lobe epilepsy, painful peripheral neuropathy, migraine aura, sensory neural hearing loss, or presbyacusis.

5. Use according to claim 1, wherein the medicament is administered following occurrence of a wind-up like event.

6. Use according to claim 1, wherein the medicament is administered prior to surgery.

7. Use of an NR2B subunit selective NMDA antagonist in the manufacture of a medicament for inhibiting neurological damage and/or preventing pain caused by central sensitization due to surgery in a mammal, **characterised in that** the medicament is administered prior to surgery.

8. Use of an NR2B subunit selective NMDA antagonist for preventing primary hyperplasia, secondary hyperplasia, primary allodynia, secondary allodynia, or other pain caused by central sensitization, in a mammal, **characterised in that** the NR2B subunit selective NMDA antagonist is administered prior to affliction with said pain.

9. Use of an NR2B subunit selective NMDA antagonist for inhibiting neurological damage and/or preventing pain caused by central sensitization due to surgery in a mammal, **characterised in that** the NR2B subunit selective NMDA antagonist is administered prior to surgery.

10. Use according to any of claims 1 to 9, wherein the NR2B subunit selective NMDA receptor antagonist is a compound of the formula 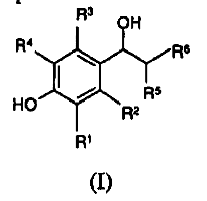 or a pharmaceutically acceptable acid addition salt thereof,
wherein
(a) R² and R⁵ are taken separately and R¹, R², R³ and R⁴ are each independently hydrogen, (C₁-C₆)alkyl, halo, CF₃, OH, or OR⁷ and R⁵ is methyl or ethyl; or
(b) R² and R⁵ are, taken together, 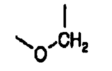
thereby forming a chroman-4-ol ring, and R¹, R³ and R⁴ are each independently hydrogen, (C₁-C₆)alkyl, halo, CF₃, OH or OR⁷;
R⁶ is 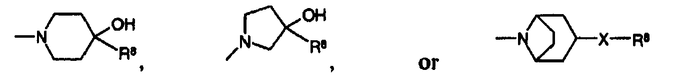 R⁷ is methyl, ethyl, isopropyl, or n-propyl;
R⁸ is phenyl optionally substituted with up to three substituents independently selected from the group consisting of (C₁-C₆)alkyl, halo and CF₃;
X is O, S or (CH₂)ₙ; and
n is 0, 1, 2 or 3.

11. Use according to any of claims 1 to 9, wherein the NR2B subunit selective NMDA antagonist is selected from:
(+)-(1S,2S)-1-(4-hydroxyphenyl)-2-(4-hydroxy-4-phenylpiperidinyl)-1-propanol; a pharmaceutically acceptable acid addition salt thereof;
(1S,2S)-1-(4-hydroxy-3-methoxyphenyl)-2-(4-hydroxy-4-phenylpiperidinyl)-1-propanol; a pharmaceutically acceptable acid addition salt thereof;
(3R,4S)-3-(4-(4-fluorophenyl)-4-hydroxypiperidin-1-yl)-chroman-4,7-diol; a pharmaceutically acceptable acid addition salt thereof; and
(IR*,2R*)-1-(4-hydroxy-3-methylphenyl)-2-(4-(4-fluorophenyl)4-hydroxypiperidin-1-yl)-propan-1-ol-mesylate.

12. Use according to any of claims 1 to 9, wherein the NR2B subunit selective NMDA antagonist is co-administered with one or more other substances selected from anaesthetics, analgesics, and anti-anxiolytics.
